(19) Europäisches Patentamt
European Patent Office
Office européen des brevets

(11) **EP 2 015 066 A1**

(12) **EUROPEAN PATENT APPLICATION**

(43) Veröffentlichungstag:
**14.01.2009 Patentblatt 2009/03**

(51) Int Cl.:
*G01N 33/48* *(2006.01)*    *G01N 33/543* *(2006.01)*

(21) Anmeldenummer: **08017911.2**

(22) Anmeldetag: **02.11.1998**

(84) Benannte Vertragsstaaten:
**AT BE CH DE ES FR GB IT LI LU NL SE**

(30) Priorität: **03.11.1997   DE 19748489**

(62) Dokumentnummer(n) der früheren Anmeldung(en) nach Art. 76 EPÜ:
**98120756.6 / 0 913 690**

(71) Anmelder: **Roche Diagnostics GmbH**
**68305 Mannheim (DE)**

(72) Erfinder:
• **Hornauer, Hans, Dr.**
  **82380 Peissenberg (DE)**
• **Sluka, Peter, Dr.**
  **82380 Peissenberg (DE)**

• **Karl, Johann, Dr.**
  **82380 Peissenberg (DE)**
• **Lenz, Helmut, Dr.**
  **82327 Tützing (DE)**
• **Mutter, Wolfgang, Dr.**
  **82347 Bernried (DE)**

(74) Vertreter: **Weiss, Wolfgang et al**
**Weickmann & Weickmann**
**Patentanwälte**
**Postfach 86 08 20**
**81635 München (DE)**

Bemerkungen:
Diese Anmeldung ist am 13-10-2008 als Teilanmeldung zu der unter INID-Code 62 erwähnten Anmeldung eingereicht worden.

(54) **Polyethylenglykol-derivatisierte Biomoleküle und deren Verwendung in heterogenen Nachweisverfahren**

(57)    Es wird die Verwendung von Polyalkylenoxid-modifizierten Reagenzien in Verfahren zum Nachweis eines Analyten oder in für solche Verfahren geeigneten Reagenzienkits offenbart.

EP 2 015 066 A1

**Beschreibung**

Die Erfindung betrifft Verfahren zum Nachweis eines Analyten in einer Probe und für solche Verfahren geeignete Reagenzienkits.

Verfahren zum Nachweis eines Analyten in einer Probe, bei denen eine Festphase verwendet wird, werden als heterogene Testformate bezeichnet. Ein Problem bei derartigen Verfahren besteht darin, daß oftmals eine unspezifische Bindung von Bestandteilen der Probe oder von Testreagenzien an die Festphase auftritt, wodurch falsche Testergebnisse erhalten werden können. Besonders häufig werden diese unspezifischen Bindungen bei Proben aus Körperflüssigkeiten, z.B. Serum oder Plasma beobachtet. Um diese unspezifischen Bindungen zu unterdrücken, besteht die Möglichkeit, den Reagenzien oberflächenaktive Substanzen wie z.B. Tween 20 zuzusetzen (WO88/07683). Bekannt ist außerdem die Funktionalisierung von metallischen Oberflächen (Whitesides et al., Science 252 (1991), 1164-1166) und von oxidischen Oberflächen (EP-A-0 664 452) mit reaktiven Polyethylenglykol-Derivaten, um die unspezifische Bindung auf der Oberfläche zu minimieren.

Dem Stand der Technik zur Verringerung der unspezifischen Bindung von oberflächenaktiven Substanzen haben den Nachteil, daß sie an der Festphase gebundene Moleküle, z.B. Festphasenrezeptoren, verdrängen und auf diese Weise die Testfunktion beeinträchtigen. Darüber hinaus werden als oberflächenaktive Substanzen in der Regel großtechnisch hergestellte Waschmitteltenside verwendet, die in ihrer Zusammensetzung heterogen sind und gelegentlich Verunreinigungen enthalten. Die daraus resultierenden Chargenschwankungen führen oft zu Störungen und nicht reproduzierbaren Ergebnissen. Darüber hinaus können empfindliche Festphasenmoleküle, z.B. Proteine, durch die oberflächenaktiven Substanzen in ihrer Struktur gestört und letztlich denaturiert werden.

Die aus dem Stand der Technik bekannte Funktionalisierung von metallischen oder oxidischen Oberflächen mit Polyethylenglykol ist einerseits auf bestimmte Arten von Oberflächen beschränkt und andererseits nicht ausreichend, um die unspezifische Bindung an eine auf der Festphasenoberfläche aufgebrachte Schicht von Biomolekülen zu verhindern.

Die der vorliegenden Erfindung zugrundeliegende Aufgabe bestand somit darin, ein neues Verfahren zur Verringerung der unspezifischen Bindung an eine Festphase beim Nachweis eines Analyten in einer Probe bereitzustellen, bei der die Nachteile des Standes der Technik mindestens teilweise vermieden werden können.

Ein erster Aspekt der vorliegenden Erfindung betrifft ein Verfahren zum Nachweis eines Analyten in einer Probe, umfassend die Schritte:

(a) Bereitstellen einer Festphase, umfassend in immobilisierter Form einen analytspezifischen Festphasenreaktanden und ein analytunspezifisches Biomolekül, das mit einem Poly ($C_2$-$C_3$)-Alkylenoxid gekoppelt ist,
(b) Inkubieren der Probe mit der Festphase und einem Testreagenz und
(c) Nachweisen des Vorhandenseins oder/und der Menge des Analyten in der Probe.

Durch Blockierung der Festphase mit einem Polyalkylenoxid-, insbesondere mit einem Polyethylenglykol-modifizierten analytunspezifischen Biomolekül konnte eine deutliche Verringerung der unspezifischen Bindung von Probenbestandteilen an die Festphase erreicht werden, ohne daß gleichzeitig die Testsensitivität signifikant beeinträchtigt wurde. Die Zugabe des Blockierungsreagenz kann während oder/und nach der Immobilisierung des Festphasenreaktanden erfolgen. Besonders bevorzugt wird eine mit einem analytspezifischen Festphasenreaktanden vorbelegte Festphase nachträglich mit einem analytunspezifischen Biomolekül blockiert.

Gute Ergebnisse wurden bei Verwendung von Festphasen erhalten, die "begrenzte Testflächen" aufweisen, d.h. mit einem Festphasenreaktanden belegte begrenzte Bereiche, die durch inerte Bereiche von weiteren Testflächen räumlich getrennt sind. Besonders bevorzugt sind flächig mit einer analytunspezifischen Vorbeschichtung, z.B. mit Streptavidin, überzogene Festphasen, die mindestens eine räumlich begrenzte Testfläche, auf der ein analytspezifischer Festphasenraktand immobilisiert ist, enthalten. Die begrenzten Testflächen haben bevorzugt einen Durchmesser von 10 μm bis 10 mm. Besonders bevorzugt sind miniaturisierte Testflächen mit einem Durchmesser von 10 μm bis 2 mm. Weiterhin bevorzugt sind Festphasen mit mehreren Testflächen, die unterschiedliche analytspezifische Festphasenreaktanden enthalten können und auch als Array-Systeme bezeichnet werden (vgl. z.B. US-Patente 5,432,099; 5,516,635 und 5,126,276). Mit diesen Array-Systemen können mehrere Analytbestimmungen gleichzeitig an einer Probe durchgeführt werden.

Die Festphase im erfindungsgemäßen Verfahren umfaßt einen beliebigen Träger, wobei nichtporöse Träger, z.B. Träger mit Kunststoff-, Glas-, Metall- oder Metalloxidoberfläche bevorzugt sind. Auch poröse Träger, wie etwa Teststreifen sind geeignet.

Auf der Festphase wird ein analytspezifischer Festphasenreaktand immobilisiert, d.h. ein Biomolekül, welches eine spezifische Wechselwirkung mit einem zu bestimmenden Analyten oder - im Falle kompetitiver Testformate - mit einem Analogon eines zu bestimmenden Analyten eingehen kann. Beispiele für analytspezifische Festphasenreaktanden sind Antikörper, Antigene, Peptide, Haptene, Nukleinsäuren, Nukleinsäureanaloga, Glykoproteine, Saccharide, Lipoproteine und andere Biomoleküle.

Die Immobilisierung des Festphasenreaktanden kann nach bekannten Methoden erfolgen, z.B. durch direkte adsorptive Bindung, durch kovalente Kopplung oder vorzugsweise durch Kopplung über hochaffine Bindepaare. Hierzu wird die Festphase zunächst mit einem ersten Partner eines hochaffinen Bindepaares belegt und daran ein Konjugat des Festphasenreaktanden mit dem zweiten Partner des Bindepaares immobilisiert. Beispiele für geeignete hochaffine Bindepaare sind Streptavidin oder Avidin/Biotin oder ein Biotinderivat (beispielsweise Desthiobiotin, Iminobiotin, Aminobiotin oder eine andere mit hoher Affinität an Streptavidin oder Avidin bindefähige Substanz), Antikörper/Hapten (beispielsweise Digoxigenin, Fluorescein etc.), Antikörer/Antigen (beispielsweise Peptid oder Polypeptid), Lectin/Zucker und Rezeptor/Ligand (beispielsweise ...). Besonders bevorzugt verwendet man als hochaffines Bindepaar Streptavidin oder Avidin/Biotin.

Das erfindungsgemäße Verfahren umfaßt vorzugsweise das Blockieren unspezifischer Bindestellen auf der bereits mit dem analytspezifischen Festphasenreaktanden belegten Festphase durch Inkubieren mit einem Alkylenoxid-modifizierten Bindemolekül, das als Blockierungssubstanz wirkt. Zeitdauer und Temperatur der Inkubation können innerhalb weiter Bereiche variiert werden, beispielhaft sind Inkubationstemperaturen von 4°C bis 40°C und Inkubationszeiten von 1 min bis 1 h.

Als Blockierungssubstanzen geeignet sind analytunspezifische bzw. inerte Biomoleküle, die an die Festphase bindefähig sind und nicht mit dem Nachweisverfahren interferieren, beispielsweise Proteine wie Albumine, unspezifische Antikörper oder Fragmente davon, oder Polysaccharide wie Dextrine etc. Die Bindung der Blockierungssubstanz an die Festphase kann über adsorptive oder kovalente Wechselwirkungen erfolgen. Bevorzugt ist jedoch eine Bindung über hochaffine Bindepaare. Insbesondere bei einer Festphase, die den Festphasenreaktanden immobilisiert über ein hochaffines Bindepaar enthält, verwendet man vorzugsweise eine Blockierungssubstanz, die den zweiten Partner des Bindepaares umfaßt, z.B. ein biotinyliertes Protein, das einen oder mehrere Polyalkylenoxidreste enthält. Alternativ ist auch die Verwendung von Blockierungssubstanzen bevorzugt, bei denen ein oder mehrere Polyalkylenoxidreste direkt an den zweiten Partner des Bindepaares gekoppelt sind.

Bevorzugte Blockierungssubstanzen sind Konjugate der allgemeinen Strukturformeln (Ia) oder (Ib):

$$P_r[-(AO_n)T]_m \qquad\qquad (Ia)$$

$$P_r\text{-}I\text{-}[-(AO_n)T]_m \qquad\qquad (Ib)$$

worin

P     ein Partner eines hochaffinen Bindepaares ist,
I     ein Biomolekül ist,
r     eine Zahl von 1 bis 10 ist,
AO   eine $(C_2\text{-}C_3)$-Alkylenoxidgruppe ist,
n     eine Zahl von 5 bis 500 ist,
T     eine Endgruppe ausgewählt aus OH, $C_1\text{-}C_4$-Alkoxy und $C_1\text{-}C_4$-Acyl ist und
m     eine Zahl von 1 bis 10 ist.

P ist vorzugsweise ein Hapten, Biotin oder ein Biotinderivat. Besonders bevorzugt ist P Biotin oder ein Biotinderivat. I ist vorzugsweise ein Polypeptid oder Saccharid. Bei Konjugaten der Formel (Ia) ist r vorzugsweise 1.

AO kann eine $(C_2\text{-}C_3)$-Alkylenoxidgruppe sein, d.h. eine Ethylenoxid- oder/und eine Propylenoxidgruppe. Vorzugsweise ist AO eine Ethylenoxidgruppe, doch auch Kombinationen von Ethylenoxid- und Propylenoxidgruppen sind geeignet. n ist vorzugsweise eine Zahl von 10 bis 250 und besonders bevorzugt 20 bis 200.

T ist eine Endgruppe (einschließlich des letzten O-Atoms der Polyalkylenoxid-Einheiten), die mit weiteren Test- und Probenkomponenten kompatibel ist, d.h. nicht signifikant störende Reaktionen eingeht. Vorzugsweise ist T eine Hydroxylgruppe, $C_1\text{-}C_4$-Alkylethergruppe, insbesondere Methoxy, oder eine $C_1\text{-}C_4$-Acylgruppe, z.B. eine Acetylgruppe. Bei Konjugaten der Strukturformel (Ia) ist m vorzugsweise 1.

Die Konjugate gemäß Strukturformel (Ia) und (Ib) werden vorzugsweise als Blockierungsreagenzien in Nachweisverfahren eingesetzt. Nach Immobilisierung auf einer Festphase sind sie vorzugsweise nicht mehr in der Lage, über die Komponente P eine hochaffine Bindung mit gelösten Biomolekülen in der Probe oder im Testreagenz einzugehen.

Ein weiterer Gegenstand der Erfindung ist eine Festphase mit einer Beschichtung, die ein oder mehrere Konjugate (Ia) oder/und (Ib) und vorzugsweise einen analytspezifischen Festphasenreaktanden enthält. Die erfindungsgemäßen Konjugate können zur Verringerung der unspezifischen Bindung an eine Festphase in einem Verfahren zum Nachweis eines Analyten eingesetzt werden, beispielweise in einem immunologischen oder einem Nukleinsäure-Hybridisierungsverfahren. Ein weiterer Gegenstand des ersten Aspekts der vorliegenden Erfindung ist ein Reagenzienkit zum Nachweis eines Analyten, der neben anderen Testkomponenten ein erfindungsgemäßes Konjugat oder eine erfindungsgemäße Festphase enthält.

In einer besonders bevorzugten Ausführungsform werden Biotin-Polyethylenglykolverbindungen verwendet, bei denen es sich um PEG-Ketten handelt, die an einem Kettenende mit einem Biotinrest funktionalisiert sind. Das andere Kettenende trägt vorzugsweise eine Hydroxyl- oder eine Methoxygruppe. Die Biotin-PEG-Konjugate werden auf eine Streptavidinfestphase nach oder gleichzeitig mit einem biotinylierten analytspezifischen Festphasenreaktanden, z.B. einem Antikörper, aufgebracht. Das Konjugat bindet auf den noch zugänglichen freien Biotinbindestellen der Streptavidinfestphase. Das nicht gebundene Biotin-PEG-Konjugat kann durch Waschen entfernt werden. Die resultierende Festphase kann in diesem Zustand getrocknet werden, ohne daß eine Beinträchtigung der Funktion auftritt. Die unspezifische Bindung einer mit einem erfindungsgemäßen Konjugat behandelten Oberfläche ist gegenüber einer unbehandelten Oberfläche oder gegenüber einer mit einer nicht-alkylenoxidmodifizierten Blockierungssubstanz behandelten Oberfläche stark vermindert. Ein weiterer Vorteil ist, daß die erfindungsgemäße Festphase auch nach dem Aufbringen des Festphasenreaktanden mit dem Blockierungskonjugat behandelt und damit mit den gewünschten Eigenschaften versehen werden kann. Bei Festphasen, welche begrenzte Testflächen und eine durchgehende Vorbeschichtung aufweisen, wird sowohl innerhalb der Testflächen als auch außerhalb dieser Testflächen (z.B. leere Streptavidinfestphase) eine deutliche Reduzierung der unspezifischen Bindung gefunden. Die Fähigkeit der Festphase zur Bindung des Analyten bleibt überraschenderweise unbeeinflußt.

Ein zweiter Aspekt der vorliegenden Erfindung ist ein Verfahren zum Nachweis eines Analyten in einer Probe umfassend die Schritte:

(a) Bereitstellen einer Festphase, auf der ein Festphasenreaktand immobilisiert ist, wobei man einen modifizierten Festphasenreaktanden verwendet, der mit einem Poly $(C_2\text{-}C_3)$-Alkylenoxid gekoppelt ist,
(b) Inkubieren der Probe mit der Festphase und einem Testreagenz und
(c) Nachweisen des Vorhandenseins oder/und der Menge des Analyten in der Probe.

Gemäß diesem zweiten Aspekt der Erfindung wird ein Polyalkylenoxidmodifizierter Festphasenreaktand auf der Festphase immobilisiert. Einerseits kann der modifizierte Festphasenreaktand ein universeller Festphasenreaktand sein d.h. ein kovalent, adsorptiv oder über ein hochaffines Bindepaar auf der Festphase immobilisierter Reaktand, der nicht spezifisch mit dem Analyten, sondern mit einem weiteren Festphasenreaktanden reagieren kann. Beispiele für universelle Festphasenreaktanden sind beispielsweise Streptavidin oder Anti-Hapten-Antikörper, die mit einem biotinylierten oder Hapten-konjugierten analytspezifischen weiteren Festphasenreaktanden reagieren können. Andererseits oder zusätzlich kann auch der analytspezifische Festphasenreaktand ein Polyalkylenoxidmodifizierter Festphasenreaktand sein.

Ein universeller modifizierter Festphasenreaktand kann beispielsweise ein Partner eines hochaffinen Bindepaares, oder ein Konjugat eines analytunspezifischen Biomoleküls mit einem Partner eines hochaffinen Bindepaares sein. Beispiele für universelle Festphasenreaktanden, die selbst den Partner eines hochaffinen Bindepaares darstellen, sind Polypeptide wie wie Streptavidin, Avidin, Hapten-spezifische Antikörper, Lectine und polymere Konjugate davon. Andererseits kann man auch ein Konjugat eines analytunspezifischen Biomoleküls mit einem Partner eines hochaffinen Bindepaares als universellen Festphasenreaktanden verwenden, beispielsweise ein inertes Polypeptid oder Polysaccharid gekoppelt mit Biotin, Biotinderivaten, Haptenen oder Zuckern.

Auch bei Verwendung eines analytspezifischen modifizierten Festphasenreaktanden ist es bevorzugt, daß dieser ein Konjugat mit einem Partner eines hochaffinen Bindepaares ist. Beispiele solcher analytspezifischer modifizierter Festphasenrezeptoren sind analytspezifische Antikörper, Antigene, Nukleinsäuren, Nukleinsäurenanaloga und Lectine.

Für den zweiten Aspekt der vorliegenden Erfindung werden in einer Ausführungsform Konjugate der allgemeinen Strukturformel (II) verwendet:

$$F[\text{-}(AO_n)T]_m \hspace{4cm} (II)$$

worin

F ein Polypeptid ausgewählt aus Lectinen, Streptavidin, Avidin und Antikörpern ist,
r eine Zahl von 1 bis 10 ist,

AO    eine $C_2$-$C_3$-Alkylenoxidgruppe ist,

n    eine Zahl von 5 bis 500 ist,

T    eine Endgruppe ausgewählt aus OH, $C_1$-$C_4$-Alkoxy und $C_1$-$C_4$-Acyl ist und

m    eine Zahl von 1 bis 10 ist,

und worin die Konjugate vorzugsweise mindestens eine Bindungsstelle aufweisen, die nach Immobilsierung der Konjugate auf einer Festphase noch eine hochaffine Bindung mit einem löslichen Reaktanden eingehen kann.

In einer weiteren bevorzugten Ausführungsform werden Konjugate der allgemeinen Strukturformel (III) verwendet:

$$P_{r'}'F_r[\text{-}(AO)_n\text{-}T]_m \qquad\qquad (III)$$

wobei

P'    ein Partner eines hochaffinen Bindepaares ist,

r'    eine Zahl von 1 bis 10 ist,

F    ein Biomolekül ist,

r    eine Zahl von 1 bis 10 ist und

AO, n, T    und m wie für Konjugate der Strukturformel (II) definiert sind.

Konjugate der Strukturformeln (II) und (III) werden vorzugsweise als universelle oder analytspezifische Festphasenreaktanden in Nachweisverfahren eingesetzt.

Der zweite Aspekt der vorliegenden Erfindung betrifft auch eine Festphase mit einer Beschichtung, die Konjugate der allgemeinen Strukturformel (II) oder/und (III) enthält. Die Konjugate können zur Verringerung der unspezifischen Bindung an eine Festphase in einem Verfahren zur Bestimmung eines Analyten beispielsweise in einem immunologischen Verfahren oder einem Nukleinsäure-Hybridisierungsverfahren eingesetzt werden. Weiterhin betrifft der zweite Aspekt der Erfindung einen Reagenzienkit zum Nachweis eines Analyten, neben anderen Testkomponenten ein Konjugat der allgemeinen Strukturformel (II) oder (III) oder eine mit einem solchen Konjugat belegte Festphase enthält.

Ein dritter Aspekt der vorliegenden Erfindung betrifft ein Verfahren zum Nachweis eines Analyten in einer Probe umfassend die Schritte:

(a) Bereitstellen einer Festphase, auf der ein analytspezifischer Festphasenreaktand immobilisiert ist,

(b) Inkubieren der Probe mit der Festphase und einem Testreagenz, wobei das Testreagenz einen analytspezifischen modifizierten löslichen Reaktanden enthält, der mit einem Poly ($C_2$-$C_3$)-Alkylenoxid gekoppelt ist, und

(c) Nachweisen des Vorhandenseins oder/und der Menge des Analyten in der Probe.

In diesem Aspekt der Erfindung wird ein modifizierter löslicher analytspezifischer Reaktand verwendet, d.h. ein mit einem zu bestimmenden Analyten oder/und Analytanalogon spezifisch bindefähiges Biomolekül. Der modifizierte lösliche Reaktand kann direkt markiert sein, d.h. eine Markierungsgruppe, z.B. eine Enzym-, Fluoreszenz- oder Elektrochemilumineszenzmarkierungsgruppe tragen. Andererseits kann der lösliche Reaktand auch indirekt markiert sein, d.h. er trägt eine mit einer nachweisbaren Markierungsgruppe reaktive Gruppe, z.B. ein Hapten, welches wiederum mit einem markierten Anti-Hapten-Antikörper reagieren kann.

Der modifizierte lösliche Reaktand wird vorzugsweise ausgewählt aus Antikörpern, Antigenen, Nukleinsäuren, Nukleinsäureanaloga und Lectinen.

Gemäß diesem dritten Aspekt der vorliegenden Erfindung werden vorzugsweise Konjugate der allgemeinen Strukturformel (IV) verwendet:

$$M_s\text{-}F''\text{-}[\text{-}(AO)_n\, T]_m \qquad\qquad (IV)$$

worin

M    eine Markierungsgruppe oder eine mit einer Markierungsgruppe reaktive Gruppe ist,

s    eine Zahl von 1 bis 10 ist,

F"   ein lösliches Biomolekül insbesondere ausgewählt aus Antikörpern, Antigenen, Nukleinsäuren, Nukleinsäureanaloga und Lectinen ist und

AO, n, T und m wie für Konjugate der Strukturformel (II) definiert sind.

Diese Konjugate können zur Verringerung der unspezifischen Bindung an eine Festphase in einem Verfahren zur Bestimmung eines Analyten, insbesondere in einem immunologischen Bestimmungsverfahren, einem Nukleinsäure-Hybridisierungsverfahren oder einem Zucker-Lectin-Bestimmungsverfahren eingesetzt werden. Weiterhin betrifft dieser dritte Aspekt der vorliegenden Erfindung einen Reagenzienkit zum Nachweis eines Analyten, der neben anderen Testkomponenten ein Konjugat der allgemeinen Formel (IV) enthält.

Gemäß einem vierten Aspekt betrifft die vorliegende Erfindung ein Verfahren zur Verringerung der unspezifischen Bindung an eine Festphase beim Nachweis eines Analyten in einer Probe, dadurch gekennzeichnet, daß man mindestens ein Reagenz verwendet, welches eine mit einem Poly ($C_2$-$C_3$)-Alkylenoxid gekoppelte Substanz enthält.

Die mit einem Poly ($C_2$-$C_3$)-Alkylenoxid gekoppelte Substanz wird vorzugsweise ausgewählt aus

(i) Blockierungssubstanzen,
(ii) universellen Festphasenreaktanden,
(iii) analytspezifischen Festphasenreaktanden und
(iv) löslichen Reaktanden

Vorzugsweise verwendet man bei dem Verfahren mehr als eine Alkylenoxidmodifizierte Klasse von Substanzen.

Ein Gegenstand dieses vierten Aspekts der Erfindung ist ein Reagenzienkit zum Nachweis eines Analyten, umfassend mindestens ein Reagenz, welches eine mit einem Poly ($C_2$-$C_3$)-Alkylenoxid gekoppelte Substanz enthält, die vorzugsweise ausgewählt wird aus einer der zuvor genannten Substanzklassen.

Weiterhin wird die Erfindung durch die nachfolgenden Beispiele erläutert.

**Beispiele**

1. Synthese eines Biotin-Polyethylenglykol (PEG)-Konjugats (MW 3499)

550 mg 1-Amino-PEG (Fa. Shearwater Polymers) wurden in 10 ml Dioxan gelöst. Zu dieser Lösung wurden 60 mg Triethylamin und anschließend 100 mg Biotin-OSu-Ester (Boehringer Mannheim) gegeben. Das Gemisch wurde 2,5 Stunden bei Raumtemperatur gerührt. Anschließend wurde das Produkt säulenchromatographisch gereinigt. Die Ausbeute betrug 30%.

**2. Synthese eines Blotin-Methoxypolyethylenglykol-Konjugats (MW 5000)**

150 mg Aminomethoxy-PEG (Fa. Sigma) wurden in 100 ml Dioxan gelöst und anschließend mit 2 g Biotin-OSu-Ester, gelöst in 60 ml DMF, versetzt. Nach der Zugabe von 40 mg Triethylamin wurde 3 Stunden bei Raumtemperatur und weitere 3 Stunden bei 70°C gerührt. Die Lösungsmittel wurden anschließend abgezogen und das Produkt säulenchromatographisch gereinigt. Die Ausbeute betrug 57%.

**3. Herstellung einer Biotin-PEG Festphase**

Auf eine Strepatvidin Festphase (p-Styrol beschichtet mit an thermisch polymerisiertes Rinderserumalbumin (RSA) gebundenem Streptavidin) wurden mittels Microdosiertechnik biotinylierte, gegen TSH gerichtete Antikörper in Form von Flächen mit einem Durchmesser von ca. 0,1 mm aufgebracht. Diese Festphase wurde nach dem Aufbringen der Antikörperflächen mit Phosphatpuffer pH 7,5, der 50 $\mu$g/ml Bi-PEG enthält, nachbehandelt. Nach 2 minütiger Inkubation wurde nachgewaschen und getrocknet.

**4. Untersuchung der unspezifischen Bindung an die Bi-PEG beschichtete Festphase**

Die nach dem in Beispiel 3 beschriebenen Verfahren hergestellte Festphase wurde mit folgendem System bewertet: Nach Inkubation mit analytfreiem Probenmaterial (p24-freies Humanserum bzw. Enzymun®-TSH 0-Standard) und anschließendem Waschschritt wurden die mit Humanserum inkubierten Festphasen mit digoxygeniliertem Nachweisreagenz (p24-Dig-Konjugat, sowie mit Anti-Human IgG-Antikörper-Dig-Konjugat) inkubiert. Die digoxygenilierten Reagenzien sind nicht spezifisch gegenüber dem Anti-TSH-Antikörper, d.h. sie enthalten keinen Analyten, stellen aber Marker für die Höhe der unspezifischen Bindung dar. Nach einem Waschschritt wurde die unspezifische Bindung durch einen fluoreszenzgefärbten, mit Anti-Dig-Antikörpern markierten Latex bestimmt. Die mittels fluoreszenzmikroskopischer Techniken erhaltenen Signale wurden mit optischer Bildauswertung quantifiziert und in Counts/sec. angegeben. Gemessen wurde die Fluoreszenzintensität innerhalb der Testflächen (mit biotinyliertem TSH-Antikörper) sowie außerhalb der Testflächen (Untergrund ohne Antikörperbeschichtung).

Tabelle 1: Ergebnisse in der Testfläche (mit TSH-Anitkörpern) in Counts/sec

| Festphase | Nachweisreagenz | | |
|---|---|---|---|
| | TSH 0-Standard | p24-Dig | <h-IgG>-Dig |
| ohne Bi-PEG | 65 | 1897 | 1612 |
| mit Bi-PEG | 31 | 740 | 1231 |

Tabelle 2: Ergebnisse im Untergrund (Streptavidin-Festphase) in Counts/sec

| Festphase | Nachweisreagenz | | |
|---|---|---|---|
| | TSH 0-Standard | p24-Dig | <h-IgG>Dig |
| ohne Bi-PEG | 51 | 766 | 654 |
| mit Bi-PEG | 17 | 136 | 140 |

In allen Fällen führte der Zusatz von Bi-PEG zu einer deutlichen Verminderung an unspezifischer Bindung auf der Festphase.

## 5. Synthese eines Streptavidin-Polyethylenglykol-Konjugats

Streptavidin und PEG-OSu wurden in Phosphatpuffer gelöst und in der jeweils gewünschten Stöchiometrie, vorzugsweise 1:1 bis 1:5 zusammengegeben. Nach 2 h Reaktion bei Raumtemperatur (?) wurde das Reaktionsgemisch gegen Phosphatpuffer mit 0,05% Natriumazid dialysiert und bei 4°C gelagert.

## 6. Herstellung einer universellen Streptavidin-PEG-Festphase

Ein Reaktionsgefäß wurde mit einer Lösung, die biotinyliertes Trägerprotein, (RSA-Biotin oder Thermo RSA-Biotin) in einer Konzentration von 100 μg/ml enthält, befüllt und 5 min bei Raumtemperatur inkubiert. Dann wurde die Lösung abgesaugt und das beschichtete Reaktionsgefäß mit Phosphatpuffer gespült und erneut abgesaugt.
Anschließend wurde Streptavidin-PEG in einer Konzentration von 50 μg/ml in Phosphatpuffer mit 1% RSA zugegeben und 15 min inkubiert. Danach wurde die Lösung abgesaugt und durch Zugabe von Phosphatpuffer mit 1% RSA und 2% Saccharose gewaschen. Nach erneutem Absaugen und Trocknen wurde die Festphase bei 4°C in luftdichter Verpackung gelagert.

## 7. Herstellung einer spezifischen Streptavidin-PEG-Festphase

Ein Reaktionsgefäß wurde mit einer Lösung, die biotinyliertes Trägerprotein in einer Konzentration von 100 μg/ml enthielt, befüllt und 5 min inkubiert. Die Lösung wurde abgesaugt, mit Phosphatpuffer gespült und erneut abgesaugt. Dann wurde Streptavidin-PEG (50μg/ml) in Phosphatpuffer mit 1 % RSA zugegeben und 15 min inkubiert. Die Lösung wurde abgesaugt, mit Phosphatpuffer gespült und erneut abgesaugt.
Dann wurde ein biotinylierter Antikörper, z.B. ein monoklonales Anti-TSH-Antikörper-Fab'$_2$-Fragment (5μg/ml) zugegeben und 15 min inkubiert. Die Lösung wurde abgesaugt, durch Zugabe von Phosphatpuffer mit 1% RSA, 2% Saccharose gespült und erneut abgesaugt. Nach Trocknen wurde die Festphase bei 4°C in luftdichter Verpackung gelagert.

## 8.1 Bewertung von Streptavidin-PEG Festphasen

Ein Reaktionsgefäß mit der Festphase aus Beispiel 6 oder 7 wurde mit einer vorverdünnten analytfreien Probe (Pferdeserum 1:1 verdünnt mit Beladepuffer 50 mM Tris/HCl pH 7,5, 0,5% RSA, 0,05% Tween 20, 0,9% NaCl) 20 min bei Raumtemperatur inkubiert. Nach Waschen wurde 20 min in Gegenwart von Signalantikörper (1 μg/ml monoklonaler Anti-TSH-Antikörper IgG-Digoxigenin-Konjugat in Beladepuffer) inkubiert und erneut gewaschen.

Nach Zugabe von Nachweisreagenz (0,01% Lösung von Fluoro-Beads mit monoklonalem Anti-Digoxigenin-Antikörper-IgG beschichtet) wurde 20 min inkubiert, gewaschen und das Fluoreszenzsignal gemessen.

Tabelle 3: Fluoreszenzleerwerte (arb. units) auf unterschiedlichen Festphasen

|  | unspezifische Festphase | spezifische Festphase |
|---|---|---|
| SA underivatisiert | 199 | 373 |
| SA-PEG (1:1) | 114 | 114 |
| SA-PEG (1:5) | (100) | (100) |

## 8.2 Unspezifische Bindung von Pufferbestandteilen

Ein Reaktionsgefäß mit der Festphase aus Beispiel 6 oder 7 wurde wie im Beispiel 8.2 beschrieben mit einer Pferdeserumprobe befüllt und gewaschen. Dann wurden 0,2 $\mu$g/ml p24-Digoxigenin in Beladepuffer zugegeben, 20 min inkubiert und gewaschen. Dann wurde Nachweisreagenz (vgl. 8.1) zugegeben, erneut 20 min inkubiert, gewaschen und das Fluoreszenzsignal gemessen. Die Ergebnisse sind in Tabelle 4 dargestellt.

Tabelle 4: unspezifische Bindung von p24-Digoxigenin (arb. units) auf unterschiedlichen Festphasen

|  | unspezifische Festphase | spezifische Festphase |
|---|---|---|
| SA underivatisiert | 691 | (>1500) |
| SA-PEG (1:1) | 260 | 660 |
| SA-PEG (1:5) | 124 | 365 |

## 8.3 Unspezifische Bindung von humanen IgG-Antikörpern

Ein Reaktionsgefäß mit den in Beispiel 6 und 7 hergestellten Festphasen wurde wie in 8.1 beschrieben, mit einer Probe befüllt und gewaschen. Die Probe war Humanserum, 1:19 mit Beladepuffer verdünnt.
Dann wurden 1,0 $\mu$g/ml monoklonaler Anti-human-IgG-Antikörper-Digoxigenin-Konjugat in Beladepuffer zugegeben und gewaschen. Anschließend wurde das Nachweisreagenz zugegeben, 20 min inkubiert, erneut gewaschen und das Fluoreszenzsignal gemessen. Die Ergebnisse sind in der nachfolgenden Tabelle 5 dargestellt.

Tabelle 5: unspezifische Bindung von Humanantikörpern (arb. units) auf unterschiedlichen Festphasen

|  | unspezifische Festphase | spezifische Festphase |
|---|---|---|
| SA underivatisiert | 2549 | (>3000) |
| SA-PEG (1:1) | 944 | 1749 |
| SA-PEG (1:5) | 515 | 834 |

## 9. Herstellung von Antikörper-PEG-Konjugaten

Die Herstellung von PEG-Antikörper-Konjugaten erfolgte wie in Beispiel 5 beschrieben, außer daß an Stelle von Streptavidin ein biotinylierter Antikörper verwendet wurde.

## 10. Herstellung von mit PEG-Antikörper-Konjugaten beschichteten Festphasen

Ein Reaktionsgefäß wurde mit einer Lösung, die mit 100 $\mu$g/ml biotinyliertes Trägerprotein (RSA-Biotin oder tRSA-Biotin) enthält, 5 min inkubiert. Dann wurde die Lösung abgesaugt, mit Phosphatpuffer gespült und erneut abgesaugt.
Anschließend wurden 50 $\mu$g/ml Streptavidin in Phosphatpuffer mit 1% RSA zugegeben und 15 min inkubiert. Diese Lösung wurde abgesaugt, mit Phosphatpuffer gespült und erneut abgesaugt. Anschließend wurden 5 $\mu$g/ml biotinylierter IgG-Antikörper, z.B. ein monoklonales Anti-TSH-Fab'$_2$-Antikörperfragment, zugegeben und 15 min inkubiert. Die Lösung wurde abgesaugt und ein Spülschritt mit Phosphatpuffer, 1 % RSA, 2% Saccharose durchgeführt. Nach erneutem Absaugen wurde das Reaktionsgefäß getrocknet und bei 4°C in luftdichter Verpackung gelagert.

## 11. Bewertung

### 11.1 Leerwert

Die Bestimmung eines Leerwerts der in Beispiel 10 hergestellten Festphase erfolgte wie in Beispiel 8.1 beschrieben. Die Ergebnisse sind in Tabelle 6 dargestellt.

Tabelle 6: Fluoreszenzleerwerte (arb. units) auf unterschiedlichen Festphasen

|                    | Signale (arb. units) |
| ------------------ | -------------------- |
| AK underivatisiert | 270                  |
| AK-PEG (1:1)       | 94                   |
| AK-PEG (1:5)       | 57                   |

### 11.2 Unspezifische Bindung von Pufferbestandteilen

Die unspezifische Bindung von Pufferbestandteilen an die in Beispiel 10 hergestellte Festphase wurde wie unter Beispiel 8.2 beschrieben bestimmt.
Die Ergebnisse sind in Tabelle 7 dargestellt.

Tabelle 7: unspezifische Bindung von p24-Digoxigenin (arb. units) auf unterschiedlichen Festphasen

|                    | spezifische Festphase |
| ------------------ | --------------------- |
| AK underivatisiert | 26658                 |
| AK-PEG (1:1)       | 23519                 |
| AK-PEG (1:5)       | 7998                  |

### 11.3 Bestimmung der unspezifischen Bindung von Human-Antikörpern (IgG)

Die Bestimmung der unspezifischen Bindung von humanen IgG-Antikörpern an die in Beispiel 10 hergestellte Festphase erfolgte wie unter Beispiel 8.3 beschrieben. Die Ergebnisse sind in Tabelle 8 dargestellt.

Tabelle 8: unspezifische Bindung von Humanantikörpern (arb. units) auf unterschiedlichen Festphasen

|                    | spezifische Festphase |
| ------------------ | --------------------- |
| AK underivatisiert | 11379                 |
| AK-PEG (1:1)       | 10475                 |
| AK-PEG (1:5)       | 4446                  |

**Beispiel 12 Durchführung eines <HIV I> Tests und Testergebnisse mit Negativproben**

Auf einen Polystyrolträger wurde auf eine Testfläche von ca. 100 $\mu$m Durchmesser ein Antigen aufgebracht, welches das gp41 des HIV-I-Virus repräsentiert. Auf die Testfläche wurden 30 $\mu$l mit Probenpuffer vorverdünnte Probe pipettiert und 20 Minuten unter Schütteln bei Raumtemperatur inkubiert. Nach Absaugen der Probe und Waschen des Testfeldes mit Waschpuffer wurden 30 $\mu$l Reagenzlösung mit einem Dig-markierten gp41-repräsentierenden HIV I-Antigen pipettiert und wiederum 20 Minuten unter Schütteln bei Raumtemperatur inkubiert. Nach Absaugen der Reagenzlösung und Waschen des Testfeldes mit Waschpuffer werden 30 $\mu$l Nachweisreagenz auf das Testfeld pipettiert. Als Nachweisreagenz dienen 100 nm große, fluoreszenzgefärbte Latexpartikel, die kovalent mit einem Anti-Dig-Antikörper beschichtet sind.

Dieses Nachweisreagenz wurde wiederum 20 Minuten unter Schütteln bei Raumtemperatur inkubiert, anschließend abgesaugt, gewaschen und trockengesaugt. Das Testfeld wurde mit einem He-Ne-Laser mit 633 nm Wellenlänge bestrahlt und die Fluoreszenz bei 670 nm Wellenlänge mit einer CCD-Kamera vermessen.

Folgende testspezifischen Reagenzien wurden verwendet:

Festphasenantigen:     Polyhapten aus gp41-Peptid
Nachweisantigen:       Polyhapten aus pg41-Peptid, Dig-markiert.

Folgende Meßwerte (Counts) wurden gemessen:

| Probe | Untergrund * [Counts] | Signal Test-feld [Counts] | Signal Test-feld - Untergrund | Cut-off-Index** |
|---|---|---|---|---|
| Negativkontrolle | 148 | 148 | 0 | 0,0 |
| Positivprobe 1 | 178 | 26435 | 26257 | 88,1 |
| Positivprobe 2 | 172 | 22908 | 22376 | 76,8 |
| Negativprobe 1 | 101 | 101 | 0 | 0,0 |
| Negativprobe 2 | 103 | 103 | 0 | 0,0 |
| Negativprobe 3 | 93 | 93 | 0 | 0,0 |
| Negativprobe 4 | 98 | 98 | 0 | 0.0 |
| Negativprobe 5 (S441) | 86 | 4401 | 4315 | 14,6 |
| Negativprobe 6 (S480) | 137 | 2690 | 2553 | 8,6 |
| Negativprobe 7 (S486) | 107 | 3833 | 3726 | 12,6 |
| Negativprobe 8 (S520) | 116 | 4331 | 4215 | 14,2 |
| * Untergrund entspricht Signal neben den Testfeldem <br> ** Cut-off-Index = $Signal_{Probe} - Signal_{Untergrund}/2 \times Signal_{Negativkontrolle}$ Cut-off-Index < 1 = negativ | | | | |

Die obige Tabelle spiegelt einen Ausschnitt aus einer Spezifitätsstudie wieder. In dieser Studie wurden ca. 240 <HIV I> negative Proben vermessen. Der Großteil der Proben (z.B. Negativproben 1-4) zeigte keine Reaktion auf den Testfeldern und war somit eindeutig negativ. Allerdings wurden 4 Proben (Negativproben 5-8) gefunden, die eine starke unspezifische Reaktion auf den Testfeldern zeigten und somit als falsch positiv detektiert wurden.

**Beispiel 13 Verbesserung der Spezifität durch PEG-derivatisiertes Festphasenantigen**

In diesem Versuch wurde ein <HIV I> Test analog Beispiel 12 durchgeführt. Im Unterschied dazu wurden auf dem identischen Testträger neben dem HIV I-Antigen zusätzlich ein identisches Antigen aufgebracht, welches im Stöchiometrieverhältnis von 1:1 mit PEG 500 derivatisiert wurde.
Folgende Meßwerte wurden erhalten:

| Probe | Untergrund* [Counts] | Polyhapten-gp41-Peptid | | Polyhapten-gp41-Peptid-PEG | |
|---|---|---|---|---|---|
| | | Counts** | COI*** | Counts** | COI*** |
| Negativ-Kontr. | 52 | 0 | 0.0 | 31 | 0.3 |
| Positiv-Kontr. | 63 | 286 | 2.0 | 8383 | 80 |
| Positivprobe 1 | 212 | 11752 | 111 | 6227 | 57.8 |
| Positivprobe 2 | 84 | 1632 | 14.9 | 3762 | 35.3 |
| S441 | 50 | 1061 | 9.7 | 79 | 0.3 |
| S480 | 53 | 871 | 7.9 | 102 | 0.5 |
| S486 | 44 | 1041 | 9.6 | 98 | 0.5 |

(fortgesetzt)

| Probe | Untergrund* [Counts] | Polyhapten-gp41-Peptid | | Polyhapten-gp41-Peptid-PEG | |
|---|---|---|---|---|---|
| | | Counts** | COI*** | Counts** | COI*** |
| S520 | 44 | 1260 | 11.7 | 84 | 0.4 |
| * Untergrund entspricht Signal neben den Testfeldem<br>** $Signal_{Probe}$-$Signal_{Untergrund}$<br>*** COI = Cut-off-Index = $Signal_{Probe}$-$Signal_{Untergrund}$/2 x $Signal_{Negativkontrolle}$ Cut-off-Index < 1 = negativ | | | | | |

Dieses Ergebnis zeigt, daß die unspezifische Bindung der Störproben in den <HIV I>-Testfläche durch Verwendung des neuen PEG-derivatisierten Antigens drastisch reduziert wird, so daß alle 4 Störproben negativ werden. Überraschenderweise kann die PEG-Derivatisierung sogar zu einer starken Erhöhung des Signals von Positivproben führen (siehe Positivkontrolle und Positivprobe 1).

### 14. Nachweis von HBs-Antigen

Auf einen Polystrolträger wurde auf eine Testfläche von ca. 100 $\mu$m Durchmesser ein monoklonaler Antikörper gegen HBs-Antigen aufgebracht. Auf eine weitere Testfläche wurde derselbe Antikörper in Form eines PEG-Konjugats (Herstellung Beispiel 9) aufgebracht. Auf die Testfläche wurden dann 30 $\mu$l mit Probenpuffer vorverdünnte Probe pipettiert und 20 min unter Schütteln in Raumtemperatur inkubiert. Nach Absaugen der Probe und Waschen der Testfläche mit Waschpuffer werden 30 $\mu$l Reagenzlösung mit Digoxigenin-markiertem Anti-HBsAg-Antikörper zupipettiert und wiederum 20 min unter Schütteln bei Raumtemperatur inkubiert. Nach Absaugen der Lösung und Waschen der Testfläche mit Waschpuffer wurden 30 $\mu$l Nachweisreagenz (Beispiel 8.3) auf die Testfläche pipettiert.

Der Nachweis erfolgte wie in Beispiel 8.1 beschrieben.

Untersucht wurden ein positiver Standard, ein negativer Standard sowie fünf Negativseren, die kein HBsAg enthalten, aber im Test dennoch signifikant positive Signale liefern, die auf analytunspezifische Wechselwirkungen mit der Festphase zurückzuführen waren. In der Tabelle 9 sind die Ergebnisse dieser Versuche aufgelistet. Es ist klar zu erkennen, daß die unspezifischen Bindungen mit PEG-derivatisierten Antikörper sehr viel niedriger ausfallen, als bei unbehandelten Antikörpern.

Tabelle 9

| | Meßsignal | |
|---|---|---|
| Probe | MAK<HBs> | MAK<HBs>PEG |
| Pos. Standard | 1080 | 960 |
| Neg. Standard | 1,3 | 1,7 |
| Negativserum 1 | 16 | 3,4 |
| Negativserum 2 | 28 | 12 |
| Negativserum 3 | 15 | 1,3 |
| Negativserum 4 | 11,5 | 2 |
| Negativserum 5 | 18 | 9 |

Im Folgenden werden bevorzugte Ausführungsformen der Erfindung zusammengefasst:

1. Verfahren zum Nachweis eines Analyten in einer Probe, umfassend die Schritte:

(a) Bereitstellen einer Festphase umfassend in immobilisierter Form einen analytspezifischen Festphasenreaktanden und ein analytunspezifisches Biomolekül, das mit einem Poly ($C_2$-$C_3$)-Alkylenoxid gekoppelt ist,
(b) Inkubieren der Probe mit der Festphase und einem Testreagenz und
(c) Nachweisen des Vorhandenseins oder/und der Menge des Analyten in der Probe.

2. Verfahren nach Punkt 1,
**dadurch gekennzeichnet,**

**daß** man eine Festphase verwendet, die mindestens eine begrenzte Testfläche aufweist.

3. Verfahren nach einem der Punkte 1 oder 2,
**dadurch gekennzeichnet,**
**daß** man eine Festphase verwendet, die mit einem ersten Partner eines hochaffinen Bindepaares belegt ist, an die ein Konjugat des analytspezifischen Festphasenreaktanden mit dem zweiten Partner des Bindepaares immobilisiert wird.

4. Verfahren nach Punkt 3,
**dadurch gekennzeichnet,**
**daß** man eine Blockierungssubstanz verwendet, die den zweiten Partner des Bindepaares umfasst.

5. Verfahren nach Punkt 3 oder 4,
**dadurch gekennzeichnet,**
**daß** man eine Blockierungssubstanz verwendet, bei der ein oder mehrere Polyalkylenoxidreste direkt an den zweiten Partner des Bindepaares gekoppelt sind.

6. Konjugate der allgemeinen Strukturformel (Ia) oder (Ib):

$$P_r[-(AO_n)T]_m \qquad\qquad (Ia)$$

$$P_r\text{-}I\text{-}[-(AO_n)T]_m \qquad\qquad (Ib)$$

worin

P   ein Partner eines hochaffinen Bindepaares ist,
I   ein inerter Träger ist,
r   eine Zahl von 1 bis 10 ist,
AO   eine $(C_2\text{-}C_3)$-Alkylenoxidgruppe ist,
n   eine Zahl von 5 bis 500 ist,
T   eine Endgruppe vorzugsweise ausgewählt aus OH, $C_1\text{-}C_4$-Alkoxy und $C_1\text{-}C_4$-Acyl ist und
m   eine Zahl von 1 bis 10 ist.

7. Konjugate nach Punkt 6,
worin P Biotin oder ein Biotinderivat ist

8. Festphase mit einer Beschichtung, die ein Konjugat nach Punkt 6 oder 7 enthält.

9. Verwendung eines Konjugats nach Punkt 6 oder 7 zur Verringerung der unspezifischen Bindung an eine Festphase in einem Verfahren zum Nachweis eines Analyten.

10. Reagenzienkit zum Nachweis eines Analyten, der neben anderen Testkomponenten ein Konjugat nach Punkt 6 oder 7 oder eine Festphase nach Punkt 8 enthält.

11. Verfahren zum Nachweis eines Analyten in einer Probe umfassend die Schritte:

(a) Bereitstellen einer Festphase, auf der ein Festphasenreaktand immobilisiert ist, wobei man einen modifizierten Festphasenreaktanden verwendet, der mit einem Poly $(C_2\text{-}C_3)$-Alkylenoxid gekoppelt ist,
(b) Inkubieren der Probe mit der Festphase und einem Testreagenz und
(c) Nachweisen des Vorhandenseins oder/und der Menge des Analyten in der Probe.

12. Konjugate der allgemeinen Strukturformel (II):

$$F[\text{-}(AO_n)T]_m \qquad\qquad (II)$$

worin

F  ein Biomolekül ist, das ein Partner eines hochhaffinen Bindepaares ist, ausgewählt aus Lectinen, Streptavidin, Avidin und Anti-Hapten-Antikörpern ist,
r  eine Zahl von 1 bis 10 ist,
AO  eine $C_2$-$C_3$-Alkylenoxidgruppe ist,
n  eine Zahl von 5 bis 500 ist,
T  eine Endgruppe ausgewählt aus OH, $C_1$-$C_4$-Alkoxy und $C_1$-$C_4$-Acyl ist und
m  eine Zahl von 1 bis 10 ist.

13. Konjugate der allgemeinen Strukturformel (III):

$$P_{r'}\text{-}F_r\text{-}(AO)_n\text{-}T]_m \qquad\qquad (III)$$

wobei

P'  ein Partner eines hochaffinen Bindepaares ist,
r'  eine Zahl von 1 bis 10 ist,
F  ein Biomolekül ist,
r  eine Zahl von 1 bis 10 ist und
AO, n, T  und m wie in Punkt 12 definiert sind.

14. Festphase mit einer Beschichtung, die ein Konjugat nach Punkt 12 oder 13 enthält.

15. Verwendung eines Konjugats nach Punkt 12 oder 13 zur Verringerung der unspezifischen Bindung an eine Festphase in einem Verfahren zur Bestimmung eines Analyten.

16. Reagenzienkit zum Nachweis eines Analyten, der neben anderen Testkomponenten ein Konjugat nach Punkt 12 oder 13 oder eine Festphase nach Punkt 14 enthält.

17. Verfahren zum Nachweis eines Analyten umfassend die Schritte:

(a) Bereitstellen einer Festphase, auf der ein analytspezifischer Rezeptor immboilisiert ist,
(b) Inkubieren der Probe mit der Festphase und einem Testreagenz, wobei das Testreagenz einen analytspezifischen modifizierten löslichen Reaktanden enthält, der mit einem Poly ($C_2$-$C_3$)-Alkylenoxid gekoppelt ist, und
(c) Nachweisen des Vorhandenseins oder/und der Menge des Analyten in der Probe.

18. Konjugate der allgemeinen Formel (IV):

$$M_s\text{-}F''\text{-}[\text{-}(AO)_n\, T]_m \qquad\qquad (IV)$$

worin

m  eine Markierungsgruppe oder eine mit einer Markierungsgruppe reaktive Gruppe ist,
s  eine Zahl von 1 bis 10 ist,
F''  ein lösliches Biomolekül ist, das spezifisch mit einem zu bestimmenden Analyten reagieren kann und
AO, n, T  und m wie in Punkt 12 definiert sind.

19. Verwendung eines Konjugats nach Punkt 18 zur Verringerung der unspezifischen Bindung an eine Festphase in einem Verfahren zur Bestimmung eines Analyten.

20. Reagenzienkit zum Nachweis eines Analyten, der neben anderen Testkomponenten ein Konjugat nach Punkt 19 enthält.

21. Verfahren zur Verringerung der unspezifischen Bindung an eine Festphase beim Nachweis eines Analyten in einer Probe,
**dadurch gekennzeichnet,**
**daß** man mindestens ein Reagenz verwendet, welches eine mit einem Poly ($C_2$-$C_3$)-Alkylenoxid gekoppelte Substanz enthält.

22. Reagenzienkit zum Nachweis eines Analyten, umfassend mindestens ein Reagenz, welches eine mit einem Poly ($C_2$-$C_3$)-Alkylenoxid gekoppelte Substanz enthält.

**Patentansprüche**

1. Verfahren zum Nachweis eines Analyten in einer Probe umfassend die Schritte:

(a) Bereitstellen einer Festphase, auf der ein Festphasenreaktant immobilisiert ist, wobei man einen modifizierten analytspezifischen Festphasenreaktanten verwendet, der mit einem Poly ($C_2$-$C_3$)-Alkylenoxid gekoppelt ist,
(b) Inkubieren der Probe mit der Festphase und einem Testreagenz und
(c) Nachweisen des Vorhandenseins oder/und der Menge des Analyten in der Probe.

2. Konjugate der allgemeinen Strukturformel (II):

$$F[\text{-}(AO_n)T]_m \qquad\qquad (II)$$

worin

F ein Biomolekül ist, das ein Partner eines hochaffinen Bindepaares ist, ausgewählt aus Lecitinen, Streptavidin, Avidin und Anti-Hapten-Antikörpern ist,
r eine Zahl von 1 bis 10 ist,
AO eine $C_2$-$C_3$-Alkylenoxidgruppe ist,
n eine Zahl von 5 bis 500 ist,
T eine Endgruppe ausgewählt aus OH, $C_1$-$C_4$-Alkoxy und $C_1$-$C_4$-Acyl ist und
m eine Zahl von 1 bis 10 ist.

3. Konjugate der allgemeinen Strukturformel (III):

$$P_{r'}\text{-}F_r\text{-}(AO)_n\text{-}T]_m \qquad\qquad (III)$$

=
wobei

P ein Partner eines hochaffinen Bindepaares ist,
r' eine Zahl von 1 bis 10 ist,
F ein Biomolekül ist,
r eine Zahl von 1 bis 10 ist und
AO, n, T und m wie in Anspruch 12 definiert sind.

4. Festphase mit einer Beschichtung, die ein Konjugat nach Anspruch 2 oder 3 enthält.

5. Verwendung eines Konjugats nach Anspruch 2 oder 3 zur Verringerung der unspezifischen Bindung an eine Festphase in einem Verfahren zur Bestimmung des Analyten.

6. Reagenzienkit zum Nachweis eines Analyten, der neben anderen Testkomponenten ein Konjugat nach Anspruch 2 oder 3 oder eine Festphase nach Anspruch 4 enthält.

7. Verfahren zum Nachweis eines Analyten umfassend die Schritte:

   (a) Bereitstellen einer Festphase auf der ein analytspezifischer Rezeptor immobilisiert ist,
   (b) Inkubieren der Probe mit der Festphase und einem Testreagenz,
   wobei das Testreagenz einen analytspezifischen modifizierten löslichen Reaktanden enthält, der mit einem Poly($C_2$-$C_3$)-Alkylenoxid gekoppelt ist, und
   (c) Nachweisen des Vorhandenseins oder/und der Menge des Analyten in der Probe.

8. Konjugate der allgemeinen Formel (IV):

$$M_s\text{-}F''\text{-}[\text{-}(AO)_n T]_m \qquad\qquad (IV)$$

worin

   m eine Markierungsgruppe oder eine mit einer Markierungsgruppe reaktive Gruppe ist,
   s eine Zahl von 1 bis 10 ist,
   F'' ein lösliches Biomolekül ist, das spezifisch mit einem zu bestimmenden Analyten reagieren kann und
   AO, n, T und m wie in Anspruch 12 definiert sind.

9. Verwendung eines Konjugats nach Anspruch 8 zur Verringerung der unspezifischen Bindung an eine Festphase in einem Verfahren zur Bestimmung eines Analyten.

10. Reagenzienkit zum Nachweis eines Analyten, der neben anderen Testkomponenten ein Konjugat nach Anspruch 9 enthält.

11. Verfahren zur Verringerung der unspezifischen Bindung an eine Festphase beim Nachweis eines Analyten in einer Probe,
   **dadurch gekennzeichnet,**
   **dass** man mindestens ein Reagenz verwendet, welches eine mit einem Poly($C_2$-$C_3$)-Alkenoxid gekoppelte Substanz enthält.

12. Reagenzienkit zum Nachweis eines Analyten, umfassend mindestens ein Reagenz, welches eine mit einem Poly($C_2$-$C_3$)-Alkylenoxid gekoppelte Substanz enthält.

Europäisches
Patentamt

European
Patent Office

Office européen
des brevets

# EUROPEAN SEARCH REPORT

Application Number

EP 08 01 7911

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 94/27137 A (UNIV UTAH RES FOUND) 24 November 1994 (1994-11-24) * page 21, lines 15-24 - page 22, lines 14-17; table 1 * * page 25 * * page 27, lines 10-14 * * page 37, lines 20-25 * ----- | 1,2,4-7, 11,12 | INV. G01N33/48 G01N33/543 |
| X | US 5 677 196 A (HERRON JAMES N [US] ET AL) 14 October 1997 (1997-10-14) * column 16, lines 20-24; table VI * ----- | 1-6,8-12 | |
| X | NISHIMURA H ET AL: "Polyethylene glycol-modified avidin: a novel agent for the selective extraction of biotinylated immune-complex in an aqueous two-phase system." JOURNAL OF BIOMATERIALS SCIENCE. POLYMER EDITION 1995, vol. 7, no. 3, 1995, pages 289-296, XP008098940 ISSN: 0920-5063 * abstract * ----- | 2,3,11, 12 | |
| X | WO 94/22429 A (LIPOSOME TECHNOLOGY INC [US]; ALLEN THERESA M [CA]; MARTIN FRANCIS J []) 13 October 1994 (1994-10-13) * page 7, paragraph 1; figure 5 * ----- | 1,3-7, 11,12 | TECHNICAL FIELDS SEARCHED (IPC) G01N |
| X | US 5 108 568 A (VAN ALSTINE JAMES M [US]) 28 April 1992 (1992-04-28) * column 7, lines 32-45 * ----- | 1,2,11, 12 | |
| X | US 4 732 863 A (TOMASI THOMAS B [US] ET AL) 22 March 1988 (1988-03-22) * column 3, lines 10-13; claims 17-19 * ----- | 8,10,12 | |
| | -/-- | | |

The present search report has been drawn up for all claims

| Place of search | Date of completion of the search | Examiner |
|---|---|---|
| The Hague | 26 November 2008 | Vadot-Van Geldre, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 08 01 7911

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 95/15979 A (NEORX CORP [US]) 15 June 1995 (1995-06-15) * page 216, lines 1-9 * * page 208, line 1 - page 210, line 6 * ----- | 2,3,6,8, 10,12 | |
| X | ENO-AMOOQUAYE E A ET AL: "Altered biodistribution of an antibody--enzyme conjugate modified with polyethylene glycol." BRITISH JOURNAL OF CANCER JUN 1996, vol. 73, no. 11, June 1996 (1996-06), pages 1323-1327, XP002084718 ISSN: 0007-0920 * page 1323, column 2, last paragraph * ----- | 3,6,8,10 | |
| X | JÄSCHKE A ET AL: "Hybridization-based affinity partitioning of nucleic acids using PEG-coupled oligonucleotides." NUCLEIC ACIDS RESEARCH 25 MAY 1994, vol. 22, no. 10, 25 May 1994 (1994-05-25), pages 1880-1884, XP002505203 ISSN: 0305-1048 * figures 1,4 * ----- | 3,6,8, 10,12 | |
| X | CUNNINGHAM-RUNDLES C ET AL: "Biological activities of polyethylene-glycol immunoglobulin conjugates. Resistance to enzymatic degradation." JOURNAL OF IMMUNOLOGICAL METHODS 10 AUG 1992, vol. 152, no. 2, 10 August 1992 (1992-08-10), pages 177-190, XP002436429 ISSN: 0022-1759 * page 180, column 1, paragraph 2 * ----- -/-- | 1,8-12 | TECHNICAL FIELDS SEARCHED (IPC) |

The present search report has been drawn up for all claims

| Place of search | Date of completion of the search | Examiner |
|---|---|---|
| The Hague | 26 November 2008 | Vadot-Van Geldre, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EUROPEAN SEARCH REPORT**

Application Number

EP 08 01 7911

Europäisches Patentamt
European Patent Office
Office européen des brevets

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | EP 0 664 452 A (BOEHRINGER MANNHEIM GMBH) 26 July 1995 (1995-07-26) * page 3, lines 17-34 * * page 4, line 55 - page 5, line 2 * * page 7, lines 21-33 * * page 11, lines 1-40 * ----- | 1-12 | |

TECHNICAL FIELDS SEARCHED (IPC)

The present search report has been drawn up for all claims

| Place of search | Date of completion of the search | Examiner |
|---|---|---|
| The Hague | 26 November 2008 | Vadot-Van Geldre, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**                    EP 08 01 7911

This annex lists the patent family members relating to the patent documents cited in the above-mentioned European search report.
The members are as contained in the European Patent Office EDP file on
The European Patent Office is in no way liable for these particulars which are merely given for the purpose of information.

26-11-2008

| Patent document cited in search report | | Publication date | Patent family member(s) | | Publication date |
|---|---|---|---|---|---|
| WO 9427137 | A | 24-11-1994 | AT | 209782 T | 15-12-2001 |
| | | | AU | 704947 B2 | 06-05-1999 |
| | | | AU | 7311694 A | 12-12-1994 |
| | | | CA | 2162996 A1 | 24-11-1994 |
| | | | DE | 69429262 D1 | 10-01-2002 |
| | | | DE | 69429262 T2 | 25-07-2002 |
| | | | DK | 700514 T3 | 25-03-2002 |
| | | | EP | 0700514 A1 | 13-03-1996 |
| | | | ES | 2169079 T3 | 01-07-2002 |
| | | | JP | 3426602 B2 | 14-07-2003 |
| | | | JP | 8510331 T | 29-10-1996 |
| US 5677196 | A | 14-10-1997 | NONE | | |
| WO 9422429 | A | 13-10-1994 | AU | 6527294 A | 24-10-1994 |
| US 5108568 | A | 28-04-1992 | NONE | | |
| US 4732863 | A | 22-03-1988 | DE | 3590392 C2 | 14-01-1993 |
| | | | DE | 3590392 T | 27-10-1988 |
| | | | GB | 2181433 A | 23-04-1987 |
| | | | JP | 5076960 B | 25-10-1993 |
| | | | JP | 62501449 T | 11-06-1987 |
| | | | WO | 8604145 A1 | 17-07-1986 |
| WO 9515979 | A | 15-06-1995 | AT | 254631 T | 15-12-2003 |
| | | | CA | 2178476 A1 | 15-06-1995 |
| | | | DE | 69433343 D1 | 24-12-2003 |
| | | | EP | 0733066 A1 | 25-09-1996 |
| | | | JP | 9506594 T | 30-06-1997 |
| EP 0664452 | A | 26-07-1995 | AT | 221660 T | 15-08-2002 |
| | | | ES | 2179853 T3 | 01-02-2003 |
| | | | JP | 3214795 B2 | 02-10-2001 |
| | | | JP | 7260790 A | 13-10-1995 |

EPO FORM P0459

For more details about this annex : see Official Journal of the European Patent Office, No. 12/82

**EP 2 015 066 A1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 8807683 A
- EP 0664452 A
- US 5432099 A
- US 5516635 A
- US 5126276 A

**In der Beschreibung aufgeführte Nicht-Patentliteratur**

- **WHITESIDES et al.** *Science,* 1991, vol. 252, 1164-1166